# EUROPEAN PATENT SPECIFICATION

(11) **EP 1 019 521 B1**
(45) Date of publication and mention of the grant of the patent: **31.05.2006**
(21) Application number: 98952675.1
(22) Date of filing: 30.09.1998
(51) Int. Cl.: C12P 19/56, C12R 1/11

(54) **A PROCESS FOR THE BIOTRANSFORMATION OF COLCHICONE COMPOUNDS INTO THE CORRESPONDING 3-GLYCOSYL DERIVATIVES**
VERFAHREN FÜR DIE BIOTRANSFORMATION VON COLCHICON-VERBINDUNGEN IN DIE ENTSPRECHENDEN 3-GLYKOSYL-DERIVATE
PROCEDE DE BIO-TRANSFORMATION DE COMPOSES DE COLCHICONE EN DERIVES CORRESPONDANTS 3-GLYCOSYL

(30) Priority: 03.10.1997 IT MI972255
(43) Date of publication of application: 19.07.2000
(73) Proprietor: INDENA S.p.A., 20139 Milano (IT)
(72) Inventor: BOMBARDELLI, Ezio, I-20141 Milano (IT); PONZONE, Cesare, I-20139 Milano (IT)
(74) Representative: Minoja, Fabrizio
(86) International application number: PCT/EP1998/006226
(87) International publication number: WO 1999/018229

(56) References cited:
- WO-A-98/15642
- US-A- 3 090 729
- US-A- 3 812 011
- CHEMICAL ABSTRACTS, vol. 119, no. 19, 8 November 1993 Columbus, Ohio, US; abstract no. 199644s, J. M. SOLET ET AL.: "Glucosylation of thiocolchicine by a cell suspension culture of Centella asiatica." page 507; XP002091355 & PHYTOCHEMISTRY, vol. 33, no. 4, 1993, pages 817-820, cited in the application

## Description

The present invention relates to the biotransformation, effected by means of selected microbial strains, of colchicinoid compounds, into the respective 3-O-glycosyl derivatives. The process of the present invention provides compounds glycosylated exclusively at C-3 of the aromatic ring A, starting from the cited colchicone compounds in high yields and purity.

The compounds obtained by the biotechnological process of the invention, particularly thiocolchicosone (3-O-glucosylthiocolchicone, i.e., with reference to formula (I), R₁ = -OCH₃ e R₂ = -SCH₃), are active principles of remarkable pharmacological importance, mainly for the preparation of new antitumor medicaments.

### DISCLOSURE OF THE INVENTION

A number of efforts have been made to obtain highly specific glycosydations of compounds of general formula (I) and related colchicinoid compounds, either by means of chemical reactions or by biotransformation.

The chemical route consists in sequences of complex, non-specific reactions which, non-selectively involving different molecular sites, lead to a mixture of glycosydated derivatives, some of which being inactive. Therefore, the conversion yields to the effective product specifically glycosydated at C-3 of the aromatic ring, are very low.

The biological approach substantially relates to the biotransformation of colchicinoid compounds, (which are indirectly related with colchicone compounds) such as colchicine and thiocolchicine, by culture of Centella Asiatica, into monoglycosydated derivatives at C-2 and at C-3 of the aromatic ring; such a transformation being therefore not highly selective and providing scarce yields and productivity (Solet, J.M., et al., Phytochemistry 33, 4, 817-820, 1993).

Other efforts to biotransform colchicinoid compounds gave simply demethylations of the methoxy groups bound to the aromatic ring (at C-2 and at C-3), anyway always characterized by limited yields and productivity and by a poor regioselectivity.

Thus, Hufford C.D. et al.. (J. Pharm. Sc., 68, 10, 1239-1242, 1979), using Streptomyces griseus and/or Streptomyces spectabilis, and Bellet P. et al.., (GB-923421, 1959), using different strains of Streptomyces and of other species of Bacteria and Fungi, tried to transform colchicine and its derivatives into the corresponding 3-demethylated derivatives. The results of these known methods confirm what stated above in connection with the non-selectivity of the microbial enzymes involved, for example at C-2, C-3 or C-10 of the alkaloid molecule. Moreover, the productivity levels of said catalytic systems are rather poor, due to the low conversion yields, the reduced substrate concentrations which can be used, and the frequent degradation of the tropolone ring.

More recently, Poulev et al.. (J. Ferment. Bioeng. 79, 1, 33-38, 1995) have obtained the specific demethylation by using bacterial microorganisms, but with still rather poor yields and productivity.

Enzyme activity from microorganisms similar to the above mentioned ones (Streptomyces, Bacillus, etc.) have been applied to the biotransformation of other compounds, such as maytansinoids (US pat. 4 361 650: Izawa, M., et al.., J. Antibiotics, 34, 12, 1587-1590, 1981). In this case also the catalysed reaction consists exclusively in a demethylation, characterized by low conversion yields and productivity.

Glycosyl transferase activities of α-amylase from Bacillus megaterium strains have been described (Brumm., P.J., et al., Starch, 43, 8, 319-323, 1991), the acceptor specificity (exclusively glucose or glucosides) being particularly high. Cyclodextrin-glucosyl transferases, produced by the same microbial source, catalyse a α-1,4-transglucosylation of rubusoside (13-O-β-D-glucosyl-steviol β-D-glucosyl ester), starting from starch. Also in this bioconversion the acceptor of the transferase reaction is the substrate glucide fraction (Darise, M., et al.., Agric. Bioel. Chem., 48, 10, 2483-2488, 1984). Cyclodextrin-glycosyl transferases were previously used for the preparation of cyclodextrins G6 and G8 from starch (Kitahata, S., Okada, S., Agric. Biol. Chem., 38, 12, 2413-2417, 1974).

These examples evidence the high substrate specificity of the glycosyl transferase activities expressed by Bacillus megaterium, which involves only glucosyl acceptors, therefore not making it possible to expect any reactions on secondary metabolites having a different, complex molecular structure, such as colchicones. In fact, no examples of the use of said microorganisms for the enzyme conversion of colchicone compounds to 3-glycosyl derivatives are known.

Now it has been found that strains of Bacillus megaterium capable of growing in the presence of high concentrations of colchicone (R₁ = -OCH₃, R₂ = -OCH₃), 3-demethyl-colchicone and respective thio derivatives, have an exceedingly high, very specific biotransformation activity of said substrates into derivatives glycosydated exclusively at C-3 of the aromatic ring. Such a transformation takes place in very short times, and is characterized by surprisingly high yields.

Therefore, the invention relates to a process for the preparation of 3-O-glycosylcolchicone compounds of formula (I): 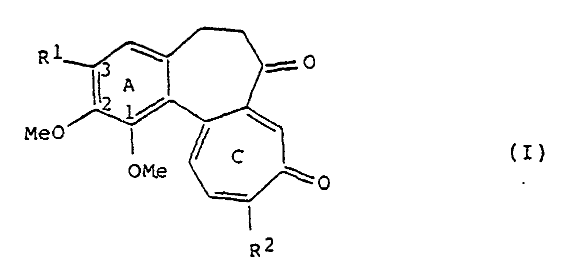 wherein R₁ is a glycosyde residue, particularly an O-glucoside residue, R₂ is C₁-C₆ alkoxy or C₁-C₆ thioalkyl, which comprises the biotransformation of compounds in which R₁ is OH or methoxy by means of Bacillus megaterium.

Bacillus megaterium is a Gram-positive spore generating bacterium with a cell diameter higher than 1.0 µm; growing aerobically on a number of culture media; catalase-positiye; hydrolysing gelatin. Strains of Bacillus megaterium which can be used according to the invention proved capable of growing satisfactorily and of keeping viable also at high concentrations of colchicone, thiocolchicone (R₁ = -OCH₃, R₂ = -SCH₃) and respective 3-demethyl derivatives (above 2. g/l), as evidenced by the examination of the growth and by microscope analysis. Congeneric species, such as Bacillus cereus, already at concentrations of substrate of 1 g/l evidence a difficulty in growing (absorbances of 10-15% of the control).

The high selectivity and efficiency of the biotransformation is surprising and unusual, as the yield levels range from 70% to 95%.

Moreover, the microorganisms used in the bioconversion are capable of maintaining permanently the catalytic activity, even in repeated fermentation steps, therefore providing the specific bioconversion in fed-batch and continuous processes. Therefore this method provides high productivity and reproducibility levels.

The marked reaction regioselectivity assures, in addition to the remarkable production yields, a high quality and purity of the resulting product, thus providing it in a 100% purity, with a simple downstream processing.

Further, important advantages are the reduced incidence of the step of purification and recovery of the product, the economicity of the process and the affidability and safety of use.

The operative sequences usable in the process of the invention comprise:
A) - Selection of cultures of Bacillus megaterium capable of growing in the presence of high concentrations of colchicone substrate, starting from natural sources or from collection strains.
B) - Selection of the isolates from A), to assay the transformation catalytic activity into the corresponding 3-O-glycosyl derivatives, by means of bioconversion assays on the specific substrates, administered in gradually increasing concentrations.
C) - Microbiological characterization of the strains selected in B).
D) - Gradual increase in the biotransformation yield, by means of a target-specific selection of the bacterial population from B).
E) - Study and optimization of the critical fermentation parameters, to optimize the biotransformation.
F) - Study and optimization of the methods for the conservation of the high-productivity cultures, to guarantee stable, homogeneous inocula for productive applications on the industrial scale.
G) - Scale-up of the process in fermenter, in batch, fed-batch and continuous processes.
H) - Working up and optimization of the methods for the downstream processing and for the recovery of the product.

Specifically, the microorganisms usable in the present invention can be selected starting from collection cultures obtained from strain deposit centers, or from soil samples of various origin, or from preselected industrial strains, by selective recovery on different agar media containing an organic nitrogen source (peptones, yeast extracts, meat extracts, asparagine, etc), a carbon source (glycerin, starch, maltose, glucose, etc.), with pH 5 to 8, preferably 6 - 7. The incubation temperature ranges from 20° to 45°C, preferably 28° - 40°C.

The ability of the culture of growing in the presence of toxic concentrations of the colchiconic substrate to be transformed is evaluated by techniques of scalar dilution and plating in parallel, on different agarized substrates, a part of which having previously been added with the colchiconic compound (e.g.: 3-demethylthiocolchicone) in concentrations from 0.1 to 3 g/l (so as to inhibit the growth of the main part of the microorganisms).

The colonies capable of growing in the described conditions are withdrawn in sterile and placed on different agarized media, to verify their purity and the homogeneity of growth.

The culture media used for the conservation of the culture are typical microbiological substrates, containing organic nitrogen sources (peptones, yeast extracts, tryptone, meat extracts, etc.), a carbon source (glucose, maltose, glycerin, etc.), at pH 5 to 8, preferably 6 - 7. The incubation temperature ranges from 20° to 45°C, preferably 28° - 40°C.

The selected microorganisms are then assayed for the capability of growing in submerged culture, in the presence of colchiconic compounds, and of transforming the latter into the corresponding 3-glycosyl derivatives.

Said assays were carried out in 100 ml flasks containing 20 ml of liquid medium, with different medium formulations, comprising one or more organic nitrogen sources (yeast extracts, peptones, tryptone, casein hydrolysates, meat extract, corn-step liquor, etc.), one or more carbon sources (glucose, glycerol, starch, saccharose, etc.), inorganic phosphorous and nitrogen sources, and inorganic salts of various ions (K⁺, Na⁺, Mg⁺⁺, Ca⁺⁺, Fe⁺⁺, Mn⁺⁺, etc.).

The culture samples can optionally be subjected to mutagenic treatments, by means of the conventional mutagenesis techniques (irradiation with UV rays, etc.) to induce mutants having a specific bioconversion activity which can be evaluated with the same procedure as above.

Culture samples from each bioconversion assay, were analyzed to evaluate the production of 3-glycosyl derivatives, by means of TLC and HPLC analysis.

The capability of the selected microorganism of transforming colchicone substrates into the respective 3-glycosyl derivatives was confirmed by means of bioconversion assays in flasks, in a 300 ml scale, in the same culture broths as used in the selection step.

The microorganisms which gave a positive response were used in tests for the optimization of the bioconversion, in different culture broths, in a 300 ml scale. The main cultural and fermentation parameters studied are: organic nitrogen sources, carbon sources, mineral salts, temperature, stirring-aeration, pH, incubation time, inoculum ratio, subculture steps, time and form of addition of the substrate to be transformed. The selected bacterial microorganisms, capable of effecting the biotransformation of the present invention, can grow on both solid and liquid culture substrates, containing one or more organic nitrogen sources, preferably yeast extract, meat extract, peptone, tryptone, casein hydrolysates, corn-steep liquor, etc.. Carbon sources useful for the growth and the biotransformation are glucose, fructose, saccharose, glycerol, malt extract, etc., preferably glucose, fructose and glycerin. The culture medium contains moreover inorganic phosphorous sources and salts of K⁺, Na⁺, Mg⁺⁺, NH₄+, etc..

The selected microorganisms can grow at temperatures from 20° to 45°C, preferably from 28° to 40°C, at pH between 5 and 8, preferably 6 - 7. In the same conditions, the considered microorganisms are capable of transforming the colchiconic compounds into the corresponding 3-glycosyl derivatives. Said transformations occur in submerged culture, in flasks incubated on a rotating shaker, with stirring from 150 to 250 rpm.

Due to the particular kinetics of the biotransformation concerned, which is related to the microbial growth, the optimum conditions for the purposes of biotransformation are the same conditions which are optimum for the growth. Therefore, culture media useful to promote a good microbial growth, such as those based on the organic and inorganic components cited above, are also useful for a good activity of biotransformation of the concerned substrate. The latter is added to the culture in the starting fermentation step, or in fractional aliquots starting from the beginning of fermentation.

The biotransformation of the invention is based on an enzyme conversion, which starts during the growth exponential phase and continues with a parallel progression to that of the growth; the maximum levels of conversion to 3-glycosyl derivative (very high: up to 95%) are reached within the first 48-72 hours, depending on the addition time of the substrate. The regioselectivity of the biotransformation is absolute: no presence of 2-glycosyl derivatives has ever been evidenced in the culture samples. The resulting products are exclusively extracellular.

The substrate to be transformed can be added in acetone or alcohol solution, in alcohol-water mixtures, in dioxane, etc. The biotransformation of the invention can be scaled up to fermenter level, keeping the culture conditions unchanged, in particular as far as culture medium, temperature and processing times are concerned. In order to obtain good growths, adequate levels of stirring-aeration are important, in particular aeration levels of 1 - 2 litres of air per litre of culture per minute (vvm), preferably of 1,5-2 vvm, are required.

The products resulting from the bioconversion are extracted from the culture broths after separation of the biomass from the liquid fraction by centrifugation and recovery of the supernatant, or microfiltration and recovery of the permeate. The culture can be treated with alcohols, in view of an optimum recovery of the product.

The purification and the recovery of the biotransformation products can be carried out using chromatographic techniques for the separation on absorption resins and elution with alcohols, preferably with methanol. The hydromethanol solutions containing the product can further be purified by extraction with lipofilic organic solvents, preferably with methylene chloride. After further treatments with mixtures of alcohols and organic solvents, the product can be obtained in the pure state from the resulting alcohol solutions by crystallization. Glucose can be replaced by other sugars, such as fructose or galactose, without causing the loss of the glycosyl transferase activity.

The following examples disclose the invention in further detail.

### EXAMPLE 1

Aliquots of cultures of Bacillus megaterium, isolated from agriculture soil, are resuspended in 20 ml of sterile saline, and subjected to a scalar dilution to a 1:10.000.000 dilution factor. The suspensions at various dilutions are plated on LB-Agar culture medium and on LB-Agar added respectively with thiocolchicone or 3-demethylthiocolchicone, to a final concentration of 2 g/l (see Table). The cultures are incubated at +28°C, for 3-4 days, in the dark. The colonies grown on the selective medium, added with the colchicone compound, are isolated and purified by means of plating on non-selective medium; said samples are incubated as above, but for a shorter time (24 hours).

Subsequently the cultures are transferred to the same agar medium, in a test-tube, and incubated as above for 24 hours.

Aliquots of cultures, selected as described, are used to inoculate 100 ml Erlenmeyer flasks containing 20 ml of culture medium ST (Table), added with thiocolchicone or 3-demethylthiocolchicone, to a 0.4 mg/ml final concentration. Said cultures are incubated overnight at 28°C, on a rotary shaker, at 200 rpm.

The transformation of the colchicone substrate is checked by analysis of aliquots of culture broths, taken every 3 - 4 hours, by TLC on silica gel, with an acetone:ethyl acetate:water 5 : 4 : 1 eluent system.

After 4 day incubation, aliquots of the cultures, which proved an evident catalytic activity towards the 3-glycosyl derivative, are recovered on plates, by means of scalar dilution as described above, for the preparation of novel inocula in test-tube. The biotransformation assay in the flask is repeated in the same conditions as above, but using markedly higher final concentrations of thiocolchicone and 3-demethylthiocolchicone (1 mg/ml). The most active single cultures (substrate conversion equal to or higher than 70%) are used for the preparation of inocula in frozen cryotubes.

**Table Formulation of the culture media**

| 1) LB-Agar | |
|---|---|
| Triptone | 10 g/l |
| Yeast extract | 5 g/l |
| NaCl | 10 g/l |
| Agar Agar | 15 g/l |
| pH 7 | |
| Sterilization: 121°C x 20' | |

| 2) Broth ST | |
|---|---|
| Glucose | 20 g/l |
| Glycerol | 10 g/l |
| Peptone | 15 g/l |
| Yeast extract | 5 g/l |
| NaCl | 3 g/l |
| NH₄Cl | 3 g/l |
| K₂HPO₄ | 8 g/l |
| KH₂PO₄ | 3 g/l |
| MgSO₄, 7H₂O | 0,5 g/l |
| pH 7 | |
| Sterilization: 121°C x 20' | |

### EXAMPLE 2

The procedure described in Example 1 is repeated, starting from Bacillus megaterium cultures, deriving from the following collection strains (Deutsche Sammlung von Mikroorganismen, Braunschweig, Germany):

| | |
|---|---|
| DSM | 90 |
| DSM | 322 |
| DSM | 333 |
| DSM | 1667 |
| DSM | 1670 |
| DSM | 1671. |

The cultures selected as in Example 1 and added with thiocolchicone (1 mg/ml) are incubated for 4 days in liquid culture: the TLC analysis detects the occurred transformation of the substrate into thiocolchicosone, with conversion yields varying from 30 to 70%.

### EXAMPLE 3

Aliquots of culture samples in test-tube, selected as described in the above example, are used to inoculate 100 ml Erlenmeyer flasks containing 20 ml of broth ST.

The broth cultures are incubated at +30°C, on a rotary shaker at 200 rpm, overnight. After incubation, the cultures are added with a glycerol sterile solution to a 20% final concentration. The cultures are then dispensed into 2 ml cryotubes and immediately immersed in liquid nitrogen.

After some days, 10% of the cultures are thawed quickly at 37°C. Aliquots of each cryotube are used to inoculate 100 ml Erlenmeyer flasks containing 20 ml of medium ST, which are subsequently incubated at +28°C, overnight (preculture), at 200 rpm. After incubation, 2 ml of each preculture are transferred in sterile into 20 ml of fresh medium ST, added with 3-demethylthiocolchicone to a 1 g/l final concentration. The biotransformation is carried out and checked in the conditions described in Example 1. The analysis confirmed that the transformation of the substrate into the 3-glycosyl derivative occurred in the quantitative terms described above (70% and higher), thus proving the catalytic stability of the frozen cultures.

Parallel controls of the broth cultures, plated on LB Agar immediately after thawing, confirm the viability, homogeneity and purity of the frozen cultures.

### EXAMPLE 4

Aliquots of cultures in cryotube, after thawing, are used to inoculate 300 ml Erlenmeyer flasks containing 50 ml of medium ST (preculture). After incubation overnight at 30°C, 250 rpm, 5 ml of preculture are transferred into 50 ml of the same medium added with 3-demethyl-thiocolchicone to a 1 g/l final concentration. The cultures are incubated for 4 days, in the same conditions as described above.

Every 4 hours, samples are taken to evaluate the growth level (measuring the absorbance at 600 nm), the thiocolchicosone production (TLC and HPLC), the sterility (on LB Agar), and for the microscope morphological examination.

TLC analysis is carried out as described in Example 1. For the HPLC analysis, 1 ml fractions of culture broths are added with 9 ml of methanol and centrifuged at 13,000 rpm for 2 minutes. The content in 3-glucosyl derivative of the supernatant is analyzed by reverse phase HPLC, with isocratic elution, by means of the water:acetonitrile 80 : 20 eluent system.

The HPLC analysis proves that, after 72-96 hours, the bioconversion of substrate to thiocolchisone is substantially completed.

The final yields to 3-glucosyl derivative, obtained by the bioconversion range from 70 to 85%.

### EXAMPLE 5

The procedure described in Example 4 is repeated, but 3-demethylthiocolchicone is added to the cultures in two fractions: 0.25 g/l at the beginning and 0.74 g/l after 24 hours.

The growth and production responses of the cultures are similar to those obtained in Example 4, with thiocolchisone yields of about 90%.

### EXAMPLE 6

One liter of ST broth in Erlenmeyer flask (inoculum) is inoculated with a cryotube culture. The flasks are incubated overnight a +30°C, 250 rpm. The inoculum is transferred in sterile into a 14 l fermenter, containing 9 l of sterile broth STL. 3-demethylthiocolchicone is added to a 1 g/l final concentration (25% at the beginning, the remainder after 20 hours). The fermentation is carried out keeping suitable levels of stirring-aeration (stirring up to 900 rpm; aeration 1 to 1.5 vvm, depending on the culture growth).

Every 2 hours, samples from the culture broths are taken and subjected to the following analysis:
- Optical density (OD) at 600 nm,
- Sterility and purity analysis of the strain (on LB Agar);
- Microscope morphology (Gram stain);
- Analysis of the thiocolchicosone content, by TLC and HPLC, as described in Examples 1 and 4, respectively.

After about 48 hour fermentation, the transformation of the substrate into thiocolchisone is almost finished. The final yield is about 85%.

### EXAMPLE 7

The procedure described in Example 6 is repeated, but after 48 hour fermentation, only 90% of the culture broths are recovered, to extract the product (fraction 1). The residual 10% is added in sterile in the fermenter with 9 l of fresh sterile medium ST containing 10 g of 3-demethylthiocolchicone. The fermentation is carried out as described in Example 6. After 48 hours, 9 l of culture broths are collected and extracted (fraction 2). The residual volume of culture broths is added sterilely with 9 more 1 of sterile fresh medium ST containing fresh 3-demethylthiocolchicone (10 g). The fermentation is carried out as above. After 48 hours the culture broth is collected completely and extracted (fraction 3). The biotransformation activity of the strain remained stable for all of the three runs, with conversion yields of about 80%.

### EXAMPLE 8

The final culture broth from the fermentation (total volume: about 27 l) is concentrated under vacuum to a soft residue and taken up with ethanol.

After separation by filtration, the water-ethanol fraction is concentrated to water, under vacuum, and purified by repeated extractions with methylene chloride. The aqueous fractions are concentrated and, after adjusting pH to 10 with sodium hydroxide, extracted with chloromethylene-ethanol mixtures.

The combined organic phases are concentrated under vacuum. The resulting suspension is added with ethanol, concentrated and left to crystallize. A second crystallization with ethanol is carried out after further redissolution steps of the solid in chloromethylene-ethanol mixtures.

## Claims

1. A process for the preparation of 3-0-glycosylcolchicone compounds of formula (I): 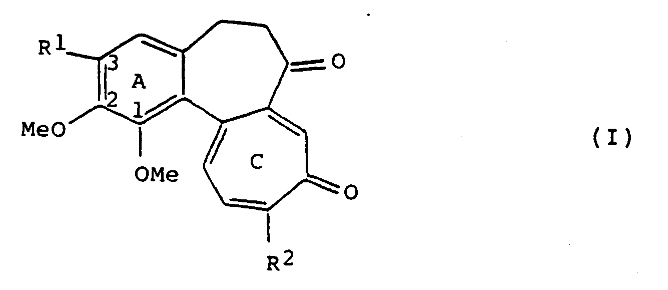 wherein R¹ is a glycosyde residue, R² is C₁-C₆ alkoxy or C₁-C₆ thioalkyl, which comprises the biotransformation of compounds in which R₁ is OH or methoxy by means of Bacillus megaterium.

2. A process for the preparation of the compounds of formula (I) in which R₁ is an 0-glucoside residue.

3. A process according to claims 1-2, wherein the Bacillus megaterium strains are selected for their capability of growing in the presence of high concentrations of colchicone substrate to be transformed.

4. A process according to claim 3 wherein said concentrations range from 0.1 to 3 g/l.

5. A process according to claims 1-4, wherein Bacillus megaterium is cultured in a solid or liquid medium.

6. A process according to claim 5, wherein said medium comprises at least one organic nitrogen source.

7. A process according to claim 6, wherein said organic nitrogen source is selected from the group consisting of meat extract, peptone, tryptone, casein hydrolysates, corn-step water.

8. A process according to claim 5, wherein the medium comprises at least one carbon source.

9. A process according to claim 8, wherein said carbon source is selected from the group consisting of glucose, fructose, glycerol.

10. A process according to claim 5, wherein said medium comprises at least one source of inorganic salts of K⁺, Na⁺, Mg⁺⁺, NH₄⁺.

11. A process according to claims 1-10, which is carried out at a pH ranging from 5 to 8.

12. A process according to claim 11, wherein said pH ranges from 6 to 7.

13. A process according to claims 1-12, which is carried out at a temperature ranging from 20° to 45°C.

14. A process according to claim 13, wherein said temperature ranges from 28° to 40°C.

15. A process according to claims 1-14, which is carried out at a maximum aeration level from 1 to 2 litres of air per litre of culture per minute (vvm).

16. A process according to claim 15 wherein said level ranges from 1.5 to 2 vvm.

## Patentansprüche

1. Verfahren zur Herstellung von 3-O-Glycosylcolchicon-Verbindungen der Formel (I): 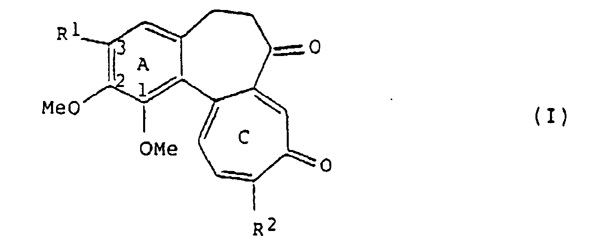 worin R¹ einen Glycosidrest darstellt, R² C₁-C₆-Alkoxy oder C₁-C₆-Thioalkyl darstellt, das die Biotransformation von Verbindungen, worin R₁ OH oder Methoxy darstellt, mit Hilfe von Bacillus megaterium umfasst.

2. Verfahren zur Herstellung von Verbindungen der Formel (I), worin R₁ einen O-Glucosidrest darstellt.

3. Verfahren nach Ansprüchen 1 bis 2, wobei die Stämme von Bacillus megaterium aufgrund ihrer Fähigkeit, in Gegenwart von hohen Konzentrationen des zu transformierenden Colchicon-Substrats zu wachsen, ausgewählt werden.

4. Verfahren nach Anspruch 3, wobei die Konzentrationen im Bereich von 0,1 bis 3 g/l vorliegen.

5. Verfahren nach Ansprüchen 1 bis 4, wobei Bacillus megaterium in einem festen oder flüssigen Medium gezüchtet wird.

6. Verfahren nach Anspruch 5, wobei das Medium mindestens eine organische Stickstoffquelle umfasst.

7. Verfahren nach Anspruch 6, wobei die organische Stickstoffquelle aus der Gruppe, bestehend aus Fleischextrakt, Pepton, Trypton, Kaseinhydrolysaten, Maisquellwasser, ausgewählt ist.

8. Verfahren nach Anspruch 5, wobei das Medium mindestens eine Kohlenstoffquelle umfasst.

9. Verfahren nach Anspruch 8, wobei die Kohlenstoffquelle aus der Gruppe, bestehend aus Glucose, Fructose, Glycerin, ausgewählt ist.

10. Verfahren nach Anspruch 5, wobei das Medium mindestens eine Quelle von anorganischen Salzen von K⁺, Na⁺, Mg⁺⁺, NH₄⁺ umfasst.

11. Verfahren nach Ansprüchen 1 bis 10, das bei einem pH-Wert im Bereich von 5 bis 8 ausgeführt wird.

12. Verfahren nach Anspruch 11, wobei der pH-Wert im Bereich von 6 bis 7 liegt.

13. Verfahren nach Ansprüchen 1 bis 12, das bei einer Temperatur im Bereich von 20° bis 45°C ausgeführt wird.

14. Verfahren nach Anspruch 13, wobei die Temperatur im Bereich von 28° bis 40°C liegt.

15. Verfahren nach Ansprüchen 1 bis 14, das bei einer maximalen Belüftungsmenge von 1 bis 2 Litern Luft pro Liter Kultur pro Minute (vvm) ausgeführt wird.

16. Verfahren nach Anspruch 15, wobei die Menge bei 1,5 bis 2 vvm liegt.

## Revendications

1. Procédé pour la préparation de composés de 3-O-glycosylcolchicone de formule (I) : 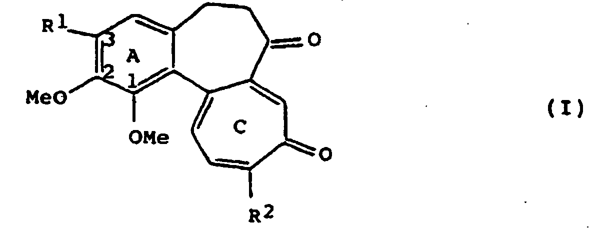 dans laquelle R¹ est un résidu glycosyde, R² est un groupe alcoxy en C₁ à C₆ ou thioalkyle en C₁ à C₆, qui comprend la biotransformation de composés dans lesquels R₁ est OH ou méthoxy au moyen de *Bacillus megaterium.*

2. Procédé pour la préparation des composés de formule (I), dans lequel R₁ est un résidu O-glucoside.

3. Procédé selon les revendications 1-2, dans lequel les souches de *Bacillus megaterium* sont choisies pour leur aptitude à se développer en présence de hautes concentrations de substrat colchicone à transformer.

4. Procédé selon la revendication 3, dans lequel lesdites concentrations vont de 0,1 à 3 g/l.

5. Procédé selon les revendications 1 à 4, dans lequel *Bacillus megaterium* est cultivé dans un milieu solide ou liquide.

6. Procédé selon la revendication 5, dans lequel ledit milieu comprend au moins une source d'azote organique.

7. Procédé selon la revendication 6, dans lequel ladite source d'azote organique est choisie dans le groupe constitué par un extrait de viande, une peptone, une tryptone, les hydrolysats de caséine, une eau de macération du maïs.

8. Procédé selon la revendication 5, dans lequel le milieu comprend au moins une source de carbone.

9. Procédé selon la revendication 8, dans lequel ladite source de carbone est choisie dans le groupe constitué par le glucose, le fructose, le glycérol.

10. Procédé selon la revendication 5, dans lequel ledit milieu comprend au moins une source de sels inorganiques de K⁺, Na⁺, Mg⁺⁺, NH₄⁺.

11. Procédé selon les revendications 1 à 10, qui est mis en oeuvre à un pH allant de 5 à 8.

12. Procédé selon la revendication 11, dans lequel ledit pH va de 6 à 7.

13. Procédé selon les revendications 1 à 12, qui est mis en oeuvre à une température allant de 20° à 45 °C.

14. Procédé selon la revendication 13, dans lequel ladite température va de 28° à 40 °C.

15. Procédé selon les revendications 1 à 14, qui est mis en oeuvre à un taux d'aération maximal de 1 à 2 litres d'air par litre de culture par minute (vvm).

16. Procédé selon la revendication 15, dans lequel ledit taux va de 1,5 à 2 vvm.
